# EUROPEAN PATENT APPLICATION

(11) **EP 2 826 444 A1**
(43) Date of publication of application: **21.01.2015**
(21) Application number: 14172139.9
(22) Date of filing: 12.06.2014
(51) Int. Cl.: A61F 2/38

(54) **Femoral component with curved central box**

(30) Priority: 16.07.2013 US 201361846854 P
(71) Applicant: Howmedica Osteonics Corp., Mahwah, NJ 07430 (US)
(72) Inventor: Logan, Scott, Ringwood, NJ 07456 (US); Chen, An, Lincoln Park, NJ New Jersey 07035 (US)
(74) Representative: Valea AB

(57) **Abstract**

A femoral component (10) for a prosthetic knee joint has a medial (14) and a lateral (12) condylar portion each having a bearing surface and a bone contacting surface. The medial and lateral condylar portions defining an opening therebetween. The opening is at least partially open to a cavity (32) surrounded by a wall (42) extending proximally from the bone contacting surface of the medial and lateral condylar portions. The wall having an inner surface defining a perimeter of the cavity and medially, laterally and posteriorly facing outer surface portions. The medially laterally and posteriorly facing surface portions being arcuate in shape.

## Description

### BACKGROUND OF THE INVENTION

The present disclosure relates to orthopedic devices and more particularly, to knee prostheses and more particularly to a prosthetic femoral component.

During articulation of a natural knee joint, flexion between the tibia and the femur takes place about a transverse axis while some relative internal/external rotation occurs between the tibia and the femur about a longitudinal axis. Such flexion and rotation is necessary to carry out a normal gate cycle. It has been established that in full extension the tibia is rotationally displaced, relative to the femur, by approximately 2-3°. As the natural knee flexes, the tibia rotates internally. According to previous studies, about 5° of rotation ordinarily occurs as the knee is articulated from 0° to 10° of flexion, thereafter, little further rotation occurs up to at least about 45° of flexion. Total rotation at 110° of flexion is approximately 20°.

Rotational stability of the natural knee is provided by the collateral and cruciate ligaments. The cruciate ligaments deter uncontrolled internal rotation within a certain range of flexion of the knee, while the collateral ligaments provide transverse stability and deter uncontrolled external rotation of the tibia. During knee replacement the anterior and posterior cruciate ligaments may be sacrificed. In the instances where the knee prosthesis is constrained to supply the stability ordinarily provided by the sacrifice ligaments, it is desirable for the knee prosthesis to mimic the natural knee as closely as possible. In these instances, the prosthesis usually is provided with tibiofemoral articular constraint to supply the stability ordinarily provided by the sacrificed anterior cruciate ligament and also a stabilizing mechanism for supplying the stability ordinarily provided by the sacrifice posterior cruciate ligament.

Typically, a femoral component is provided with a housing disposed in a receptacle formed in the trochlear groove area of the femur. The housing provides an internal cavity defined by medial and lateral sidewalls extending from the proximal bone contacting surface of the femoral condyles of the prosthesis. The cavity in the housing typically receives a post formed on the tibial bearing component which post extends proximally. The combination of the post and the cavity within the housing provide the necessary constraint between the femur and tibia during knee flexion so that the stability originally provided by the cruciate ligaments is maintained.

The housing is typically a box having planar sidewalls. This requires the central area of the femur to have a box-like recess formed therein by the removal of bone.

### BRIEF SUMMARY OF THE INVENTION

The present invention relates to a femoral component for a prosthetic knee joint having a medial and a lateral condylar portion each having a bearing surface and a bone contacting surface. The medial and lateral condylar portions define an opening therebetween. The opening is at least partially open to a cavity surrounded by a wall extending proximally from the bone contacting surface of the medial and lateral condylar portions. The wall of the cavity has an inner surface defining a perimeter of the cavity and medially, laterally and posteriorly facing outer surface portions. The medially laterally and posteriorly facing surface portions being arcuate in shape. The arcuate medially, laterally and posterior facing outer surfaces may form a cylinder. The cylindrical medially, laterally and posteriorly facing surface may form part of a single diameter cylinder. The single cylinder has a central axis intermediate anteriorly and posteriorly facing bearing surfaces of the femoral component and may be coaxial with the anatomic axis of the femur. The cylinder central axis may lie in a sagittal plate bisecting the femoral component. The central axis of the cylinder may be located closer to the anteriorly facing bearing surface than the posteriorly facing bearing surface. The cavity inner surface perimeter may have medially, laterally and posteriorly facing proximal surfaces which define a planar proximally facing surface extending therebetween. The cavity may be open in the proximal direction between the planar proximally facing surfaces. The cavity may also have planar medially and laterally facing inner surfaces.

The femoral component for a prosthetic knee joint which has a medial and a lateral condyle having a bone contacting surface defines a space therebetween capable of receiving a proximally extending post of a tibial component. A wall extends proximally from the bone contacting surfaces on a medial side of the lateral condyle and a lateral side of the medial condyle. The wall has a part-cylindrical outer bone contacting surfaces extending proximally from the bone contacting surface. The medially, laterally and posterior facing outer surfaces may be completely cylindrical. The cylindrical medially, laterally and posteriorly facing surface form part of a single cylinder. The single cylinder has a central axis intermediate anteriorly and posteriorly facing bearing surfaces of the femoral component. The wall defines a cavity having an inner surface. The wall has medially, laterally and posteriorly facing proximal surfaces extending between the inner and outer surfaces which define a planar proximally facing surface extending therebetween. The cavity may open in the proximal direction between the planar proximally facing surfaces. The cavity may have planar medially and laterally facing inner surfaces.

As used herein when referring to bones or other parts of the body, the term "proximal" means close to the heart and the term "distal" means more distant from the heart. The term "inferior" means toward the feet and the term "superior" means toward the head. The term "anterior" means toward the front part or the face and the term "posterior" means toward the back of the body. The term "medial" means toward the midline of the body and the term "lateral" means away from the midline of the body.

### BRIEF DESCRIPTION OF THE DRAWINGS

FIG. 1 is a isometric view of the femoral component viewed from the bone contacting side thereof;
FIG. 2 is a top view of the proximally facing surface of the femoral component of FIG. 1;
FIG. 3 is a partial enlarged view of the arcuate central box area of the component of FIG. 2;
FIG. 4 is an elevation view of the femoral component of FIG. 2;
FIG. 5 is an isometric view of the femoral component of FIG. 1 mounted on a tibial tray with a post of the tibial tray extending into the cavity of the femoral component box; and
FIG. 6 is a posterior isometric view of the femoral component and tray shown in FIG. 5.

### DETAILED DESCRIPTION

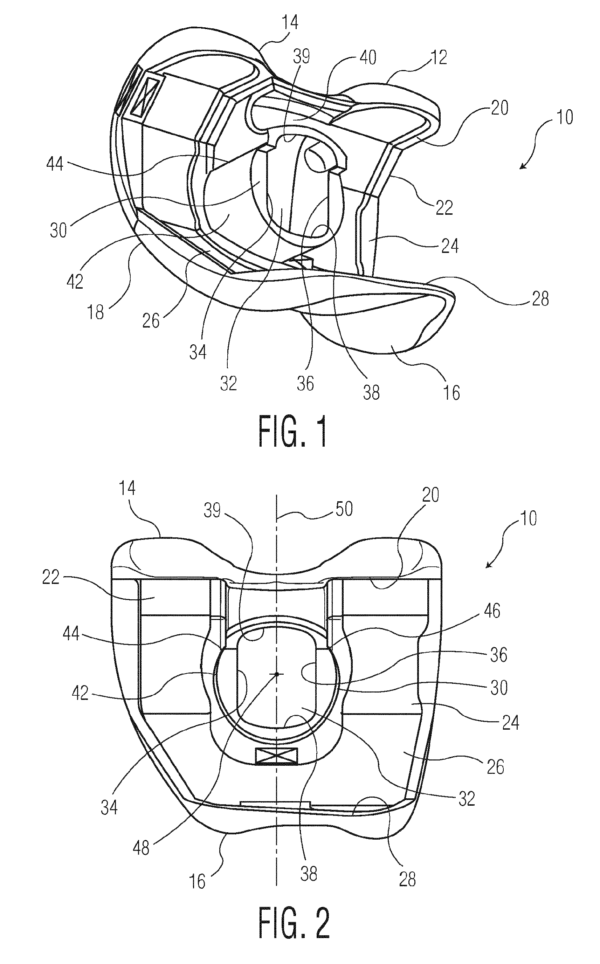

Referring to FIG. 1 there is shown an isometric view of a femoral component generally denoted as 10. The femoral component 10 includes posterior condyles 12 and 14 and an anterior surface 16. Femoral component 10 includes a bearing surface 18 designed to articulate on a tibial component preferably made of ultrahigh molecular weight polyethylene (UHMWPE).

Femoral component 10 has a bone contacting surface comprising a posterior condylar contact surface 20, a posterior chamfer surface 22, a distal surface 24, an anterior chamfer surface 26 and an anterior surface 28. These surfaces extend across the implant from the medial side to the lateral side and are designed to contact a prepared distal femur having resected surfaces corresponding to the bone contacting surfaces of femoral component 10. Femoral component 10 also includes a stabilizing box 30 defining a cavity 32 having preferably planar medial and lateral side walls 34 and 36 and an anterior wall 38. A cam surface 40 is formed at the posterior end of the implant 10 and is adapted to engage a stabilizing post on a tibial component as will be described below. As is well known, the cam surface provides for the anterior-posterior movement of the femoral component with respect to the tibial component as the knee goes from flexion to extension. The stabilizing box 30 includes an arcuate preferably cylindrical wall 42 which extends from a posterior end 44 of stabilizer box 30 on the medial side to a similar location on the lateral side.

Referring to FIG. 2 there is shown a top view of FIG. 1 viewing the bone contacting surface from the proximal direction. It can be seen that box 30 is bisected by an axis 50 in the sagittal plane which is parallel to walls 34 and 36 of inner cavity 32. Arcuate or cylindrical surface 42 extends from point 44 to point 46 when the medial and lateral sides of the implant. Preferably surface 42 is a cylinder with a single radius from center 48 between points 44 and 46. As discussed above, the use of a arcuate or cylindrical surface for box 30 results in less bone being removed during preparation of the femur for receiving femoral component 10 as well as eliminating various corners in the prepared bone that may weaken the resected femur. Also shown in FIG. 2 are the femoral component anterior chamfer surface 26, the distal surface 24 and the posterior chamfer surface 22. As can be easily seen in the top view, the anterior and posterior surfaces 38 and 39 of the cavity 32 are preferably arcuate and connect side walls 34 and 36.

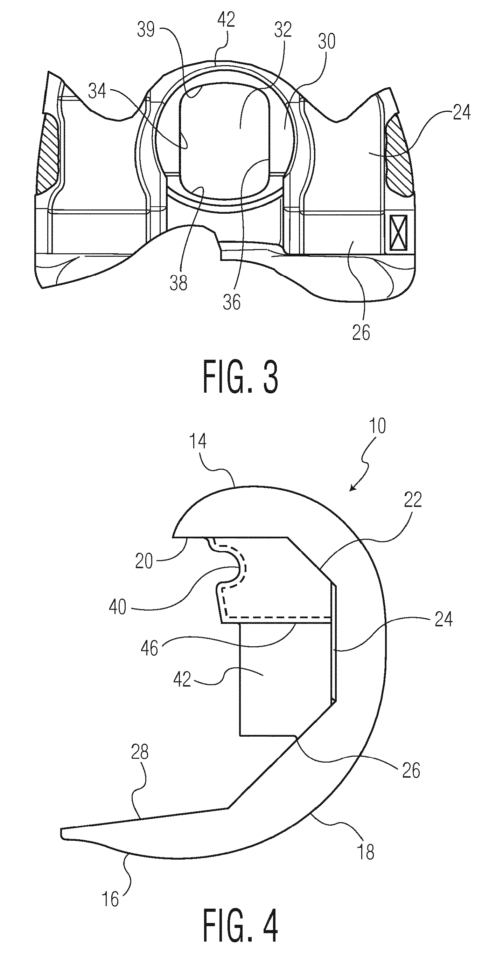

Referring to FIG. 3, there is shown an enlarged view of the arcuate or cylindrical outer surface 42 of FIG. 2 and walls 34 and 36 defining the medial and lateral side walls of cavity 32. Also shown in FIG. 3 are anterior and posterior end walls 38 and 39.

Referring to FIG. 4, there is an elevation view of femoral component 10 shown in FIG. 2 with anterior flange 16 and posterior flange 14 and bone contacting surfaces 22, the distal bone contacting surface 24, the anterior chamfer bone contacting surface 26 and the anterior bone contacting surface 28. Cam surface 40 is shown as well as cylindrical surface 42.

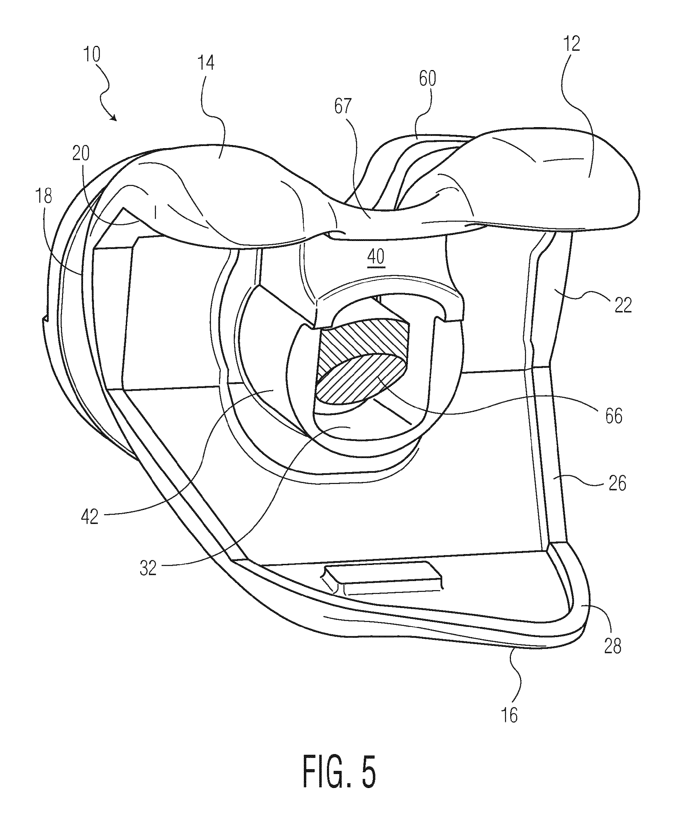

Referring to FIG. 5, there is shown an isometric view of femoral component 10 mounted on a UHMWPE tibial component 60 which includes medial and lateral condyles and a stabilizing post 66 which is received within cavity 32. As femoral component 10 rotates from flexion to extension, the total joint is stabilized by the interaction of post 66 and cam surface 40 as is well known.

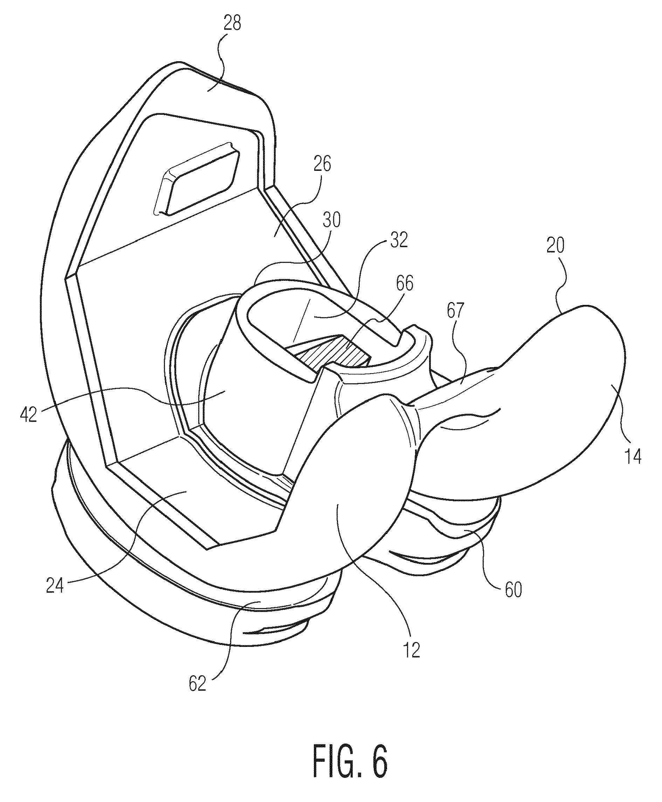

Referring to FIG. 6, there is an alternate isometric view showing the component of FIG. 5 with the post of the tibial component extending within cavity 32. Also shown is a portion 66 which forms the proximal posterior end of the intracondylar recess 67 typical in many femoral components. Recess 67 bears against the patella as the knee flexes.

The method of implantation of femoral component 10 will now be described in brief. As is typical, the distal femur is resected typically first providing a distal femoral cut to match distal surface 24 of the femoral component and then the posterior, posterior chamfer, anterior chamfer and anterior cuts were made which conform to the shape of the corresponding bone contacting surfaces 28, 22, 26 and 28 of femoral component 10. In typical stabilized femoral component implantation, a box cutter which is generally a pair of parallel planar chisel-like cutting surfaces is used to form a recess in the femur which has planar medial and lateral and anterior sides. Once this piece of bone is removed, a femoral component with an integral box with planar, medial and lateral sides is implanted on the prepared distal femur or, in some cases, a stabilizing box having the planar, medial lateral and anterior surfaces is attached to a femoral component and then implanted. This would be a modular design where the stabilizing box may or may not be used depending on which ligaments are cut.

Use of the femoral component 10 of the present invention is simplified because of the cylindrical surface 42 which can be inserted into a cylindrical counterbore in the distal femur. This counterbore can be easily formed by first using a drill or mill to form a cylindrical counter bore in the distal femur and then removing bone from the posterior side of the counter bore to accommodate the posterior cam area of the femoral component. This procedure for implanting femoral component 10 removes less bone and provides for a better distribution of the stresses within the distal femur because of the smooth arcuate bone contacting surface 42 as opposed to planar medial and lateral surfaces with small radius corners.

While the femoral component 10 shown as being unitary or a one-piece design, as indicated above, it may be a modular design wherein the box 30 may be attached to the femoral component such as by a pair of screws engaging medial and lateral threaded counter bores in the bone contacting surface of the medial and lateral condyles of the femoral component.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A femoral component for a prosthetic knee joint comprising:
a medial and a lateral condylar portion each having a bearing surface and a bone contacting surface, the medial and lateral condylar portions defining an opening therebetween, the opening at least partially open to a cavity surrounded by a wall extending proximally from the bone contacting surface of the medial and lateral condylar portions, the wall having an inner surface defining a perimeter of the cavity and medially, laterally and posteriorly facing outer surface portions, the medially laterally and posteriorly facing surface portions being arcuate in shape.

2. The femoral component as set forth in claim 1 wherein the arcuate medially, laterally and posterior facing outer surfaces are cylindrical.

3. The femoral component as set forth in claim 2 wherein the cylindrical medially, laterally and posteriorly facing surface form part of a single cylinder.

4. The femoral component as set forth in claim 3 wherein the single cylinder has a central axis intermediate anteriorly and posteriorly facing bearing surfaces of the femoral component.

5. The femoral component as set forth in claim 4 wherein the cylinder central axis lies in a sagittal plate bisecting the femoral component.

6. The femoral component as set forth in claim 4 wherein the central axis of the cylinder is located closer to the anteriorly facing bearing surface than the posteriorly facing bearing surface.

7. The femoral component as set forth in any one of claims 1 to 6 wherein the cavity inner surface perimeter has medially, laterally and posteriorly facing proximal surfaces which define a planar proximally facing surface extending therebetween.

8. The femoral component as set forth in claim 7 wherein the cavity is open in the proximal direction between the planar proximally facing surfaces.

9. The femoral component as set forth in any one of claims 1 to 8 wherein the cavity has planar medially and laterally facing surfaces.

10. The femoral component as set forth in any one of claims 2-9 wherein the central axis of the cylinder is located closer to an anteriorly facing bearing surface than to a posteriorly facing bearing surface of the medial and lateral condyles.

11. The femoral component as set forth in claim 10 wherein the wall defines a cavity having inner surfaces, the wall has medially, laterally and posteriorly facing proximal surfaces extending between the inner and outer surfaces which define a planar proximally facing surface extending therebetween.

12. The femoral component as set forth in claim 11 wherein the cavity has planar medially and laterally facing inner surfaces.
